# EUROPEAN PATENT SPECIFICATION

(11) **EP 2 198 014 B1**
(45) Date of publication and mention of the grant of the patent: **06.07.2011**
(21) Application number: 08805044.8
(22) Date of filing: 03.10.2008
(51) Int. Cl.: C12N 5/0783

(54) **NON-CONVENTIONAL NKT CELLS FOR USE IN CANCER THERAPY**
NICHT HERKÖMMLICHE NKT-ZELLEN ZUR VERWENDUNG IN DER KREBSTHERAPIE
CELLULES NKT NON CONVENTIONNELLES EN VUE D'UNE UTILISATION EN CANCÉROTHÉRAPIE

(30) Priority: 05.10.2007 EP 07301434
(43) Date of publication of application: 23.06.2010
(73) Proprietor: Inserm (Institut National de la Santé et de la Recherche Scientifique), 75654 Paris Cedex 13 (FR)
(72) Inventor: MARIE, Julien, F-69007 Lyon (FR)
(74) Representative: Blot, Philippe Robert Emile
(86) International application number: PCT/EP2008/063285
(87) International publication number: WO 2009/043931

(56) References cited:
- US-A1- 2006 216 316
- MARIE JULIEN C ET AL: "Cellular mechanisms of fatal early-onset autoimmunity in mice with the T cell-specific targeting of transforming growth factor-beta receptor" IMMUNITY, vol. 25, no. 3, September 2006 (2006-09), pages 441-454, XP002462652 ISSN: 1074-7613 cited in the application
- LI MING O ET AL: "Transforming growth factor-beta controls development, homeostasis, and tolerance of T cells by regulatory T cell-dependent and -independent mechanisms" IMMUNITY, vol. 25, no. 3, September 2006 (2006-09), pages 455-471, XP002462653 ISSN: 1074-7613
- HORWITZ DAVID A: "Transforming growth factor-beta: Taking control of T cells' life and death" IMMUNITY, vol. 25, no. 3, September 2006 (2006-09), pages 399-401, XP002462654 ISSN: 1074-7613
- SWANN J B ET AL: "CD1-restricted T cells and tumor immunity." CURRENT TOPICS IN MICROBIOLOGY AND IMMUNOLOGY 2007, vol. 314, 2007, pages 293-323, XP009093807 ISSN: 0070-217X

## Description

The invention relates to the field of cell therapy. More precisely, the invention provides a subset of NKT cells for controlling tumor growth.

### BACKGROUND OF THE INVENTION

The Transforming Growth Factor Beta (TGFβ) is a secreted polypeptide cytokine belonging to a wide family that includes TGFβs, BMPs and activins. The three forms of TGFβ, namely TGFβ1, TGFβ2, and TGFβ3, secreted at different levels by most cell types, serve as positive and negative regulators of differentiation and proliferative programs (Letterio and Roberts, 1998, Gorelik and Flavell 2000). Animals deficient in either TGFβ2 or TGFβ3 die during embryogenesis, while the deprivation of TGFβ1 is associated with myocardiopathy and immune system disorders leading to the death of the animals by three weeks of age. TGFβ signaling involves the engagement of a widely expressed receptor composed of two subunits, TβRI and TβRII. Upon binding to its receptor, TGFβ induces the kinase activity of the intracellular domain of the common TβRII subunit, which in turn phosphorylates the kinase domain of the TRI subunit. Upon another round of catalytic phosphorylation, the latter facilitates Smad-dependent regulation of gene transcription and activates several other signaling pathways in a Smad-independent manner (MAPK, Wnt/beta-Catenin, PI3K, etc) (Derynck and Zhang, 2003). Once phosphorylated by TRI receptor, Receptor-associated Smad proteins (R-Smad), Smad2 and Smad3, interact with Common-Smad (Co-Smad) Smad4. The complex Smad2/3/4 then accumulates within the nucleus, binds to DNA and activates the transcription of target genes (Massagué 2005). Recently, Transcriptional Intermediary Factor 1 gamma (TIF1γ) has been described as a selective binder of phosphorylated Smad2/3, assuring a Smad4 independent signaling pathway (He et al. 2006).

TGFβ is a key factor for tumor transformation. Indeed, TGFβ signaling response is impaired in the vast majority of tumors as a result of mutations affecting TGFβ receptors, Smad signal transducers, or by selective loss of downstream cytostatic, differentiative, or apoptotic gene responses (Levy L, et al. 2006). In animal models, disruption of TGFβ signaling often results in neoplasia (Chang H, et al 2002). During tumor progression, TGFβ is considered as a "two-edge sword". At the early stages of tumorigenesis, it acts directly on cancer cells to suppress tumor outgrowth. As the tumor progresses, TGFβ stimulates tumor progression by its activity both on cancer cells per se (for instance in Epithelial-to-Mesenchymal Transition or EMT) and on non-malignant stromal cells, such as in angiogenesis or modification of extracellular matrix allowing invasion and metastasis. Another pro-oncogenic effect of TGFβ, largely overlooked in numerous oncology studies, is its capacity to suppress the immune system and thus to allow tumor cells to escape anticancer T lymphocyte immunity.

TGFβ causes a direct impairment of T cell proliferation and activation, by repressing their functions and their capacity to eliminate potentially dangerous cells (Letterio et Roberts 1998, Gorelik et Flavell 2000). When challenged with TGFβ producing tumors, mice whose T cells have been rendered resistant to TGFβ by expression of a dominant negative TGFβ receptor transgene are able to mount an immune response, eliminate the tumor, and survive. Moreover adoptive transfer experiments have demonstrated that cytotoxic T lymphocytes (CTL) are centrally responsible for the tumor clearance process. Activated CTLs typically utilize two major contact-dependent pathways to kill targets. One is the granule exocytosis pathway. When an activated CTL recognizes a tumor cell, the membrane-pore-forming protein, perforin, mediates delivery of the apoptosis-inducing proteases Granzyme A or B into the target cell. The second contact dependent mechanism, the Fas-FasL ligand (FasL) pathways, actives target cell death via cytochrome c release and the activation of caspases. Additionally, soluble mediators including TNF-α and interferon γ (IFNγ) are secreted and induce target cell cytotoxicity. It was recently demonstrated that the exclusive and specific deletion of TβRII in T cells, or TGFβ neutralization, *in vivo* result in the acquisition of a cytotoxic activity program in T cells by over expression of FasL, granzyme A and B, perforin, TNF-α and IFNγ (Thomas et al 2005, Marie et al 2006). More precisely, the absence of TGFβ signaling in T cells induces the generation of a T cell subset expressing also natural killer cell (NK) markers (KIR, KAR, Karap, NKG2D...). Unlike the conventional Natural Killer T cells (NKT), which recognize CD1d-lipid complexes, this cell subset recognizes, like T cells, MHC-peptide complexes (Marie et al 2006).

### SUMMARY OF THE INVENTION

The inventors have now shown that this NKT cell subset, named non-conventional NKT (nc-NKT), has a unique capacity to control tumor growth.

It is herein described CD1d-independent NKT cells, for use in a method for treating tumors in patients.

A subject of the invention is thus the use of CD1d-independent NKT cells, for the preparation of a pharmaceutical composition for treating a tumor in a patient.

Said CD1d-independent NKT cells may be obtained by (a) culturing a population of T cells under conditions which suppress TGFβ signalling pathway, and (b) selecting the cells which exhibit at least one NK marker

The pharmaceutical composition advantageously controls tumor growth.

### LEGENDS TO THE FIGURES

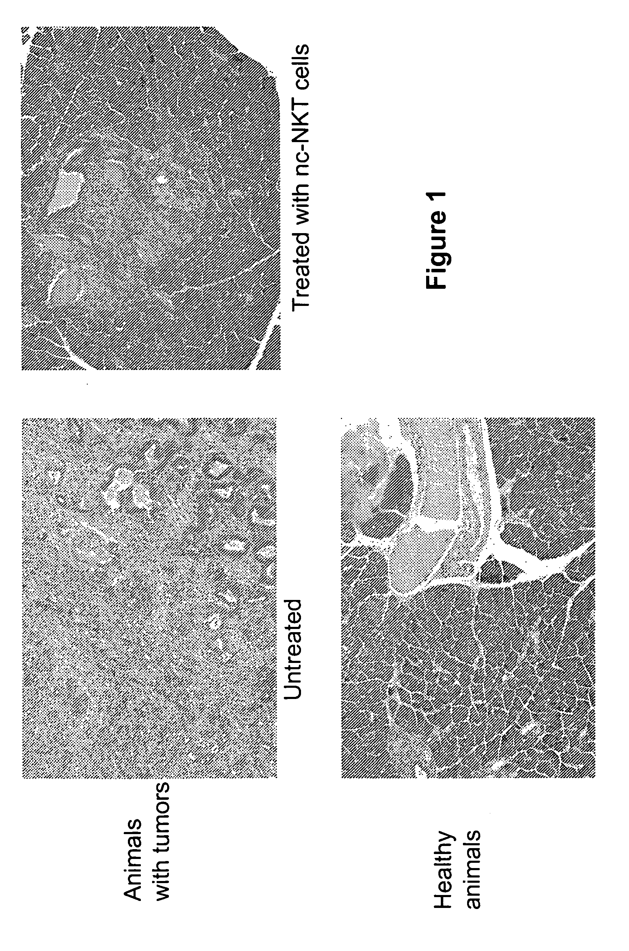

Figure 1 shows analysis of pancreatic tissue. PDX-CRE⁺ Kras^{+/-} P16^{-/-} mice of 3 weeks, which develop massive pancreatic tumors when they are 2 week old, received a single IP injection of 2x10⁵ nc-NKT cells ("treated with nc-NKT cells") or of medium ("untreated"). Three weeks later a histological analysis was performed. Five animals were used per group. Healthy animals are shown as a comparison. Observation shows that, after injection, the structure of the pancreatic channels comes back substantially to normal.

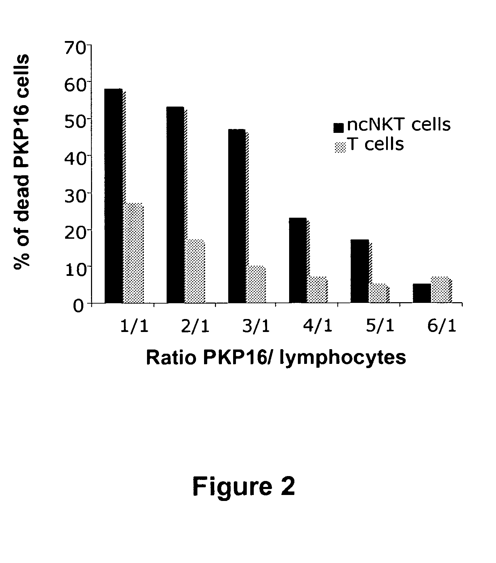

Figure 2 is a graph representing % of survival of PKP16 cells after culture with different concentrations of either nc-NKT cells or of T-lymphocytes.

### DETAILED DESCRIPTION OF THE INVENTION

### Definitions

"CD1d-independent NKT cells" consist of a subset of T cells which express a T cell receptor (TCR), NK markers, do not recognize CD1d-lipid complexes but recognize MHC-peptide complexes.

In normal mice, this population represents less than 2% of the total T cells from the spleen and reaches 20% when it is deprived of TGFβ signalling.

This T cell subset, described in Marie et al 2006, can be generated from naive T cells after their activation and blocking of TGF-β signalling pathway. nc-NKT cells, in addition to NK markers, express high amount of FasL, perforin, granzymes, IFN-γ and TNF-α, and are highly cytotoxic.

The patient to treat may be any mammal (including humans, primates, horses, cattle, rodents, sheep, feline, canine, etc), of any age or sex. Preferably the patient is a human being, who can be a male or a female, an adult, or a child.

In the context of the invention, the term "treating" or "treatment" means reversing, alleviating, or inhibiting the progress or invasiveness of the tumor.

### Production of the nc-NKT cells

In a preferred embodiment, the nc-NKT cells may be obtained by differentiation from T lymphocytes. They are preferably of human origin, preferably from the patient or from healthy donors. They may be prepared from any biological sample, such as blood, bone marrow or cord blood. Biological samples may be normal or pathological.

For instance, Human peripheral blood mononuclear cells (PBMCs) can be isolated by density gradient centrifugation. Monocytes are then depleted by adhesion and differential centrifugation to obtain peripheral blood lymphocytes (PBLs). To obtain purified T cells, PBMCs are depleted of B cells and monocytes. Cell populations can be purified using an AutoMACS magnetic cell sorter and magnetic beads (Miltenyi Biotec) and/or using a FACSVantage, ARIA cell sorter (BD Biosciences). The purity of the population is preferably superior to 90%, more preferably superior to 95%, even more preferably superior to 97%, which can be verified by flow cytometry, e.g. using anti-human CD3 conjugated antibody.

CD1d-independent NKT cells are then obtained by
a. culturing the population of T cells under conditions which suppress TGFβ signalling pathway, and
b. selecting the cells which exhibit at least one NK marker.

The culture medium employed in step (a) can be a conventional medium, so long as it does not contain or is not supplemented in TGFβ.

AIM V® (GIBCO), a serum-free medium liquid therapeutic grade, can be employed for therapeutic purposes.

In an analysis of gene expression, T cells can be cultured in RPMI 1640 supplemented with 10% FCS, 200 mM L-glutamine, 1 mM sodium pyruvate, 10 mM Hepes, 100 U/ml penicillin/streptomycin, and 5 x 10⁻⁵ M 2-mercaptoethanol (RP-10).

The population of T cells in (a) are cultured in the presence of an inhibitors of TGFβ signalling, e.g. an anti-TGFβ antibody or a TGFβ inhibitor. Examples of inhibitors of TGFβ signalling are reviewed in Muller and Scherle, 2006, or Kaminska et al, 2005.

Anti-TGFβ antibodies include Lerdelimumb and Metelimumab (Cambridge Antibody Technology).

The term "TGFβ inhibitor" includes small-molecules which inhibit TGFb receptor type I, and or TGFB signalling pathways, such as SB-505214, which is 2-(5-benzo[1,3]dioxol-5-yl-2-tert-butyl-3H-imidazol-4-yl)-6-methylpyridine hydrochloride (GlaxoSmithKline), SD-208 (Johnson&Johnson/Scios), or LYS80276 (Lilly).

Anti-TGFβ antisense oligonucleotide can further be used, such as AP-12009 or AP-11014 (Antisense Pharma).

The time period in which the T cells are cultured is, at least in part, a function of the culture medium and the matrix. Typically, culture step (a) lasts about one week.

Culture of the cells is stopped when they have sufficiently proliferated and have differentiated in nc-NKT cells.

nc-NKT cells are then sorted out, e.g. by selecting the T cells which exhibit NK markers, e.g. NKG2A, NKG2C, NKG2E, NKG2D, CD94, or DX5 and/or which do not recognize GalCer-CD1d tetramers.

For example, immunological procedures can be employed to recover the desired cell type, e.g. by using flow cytometry with anti-marker antibodies linked to magnetic beads.

### Therapy

The pharmaceutical composition containing the CD1d-independent NKT cells is preferably in a suitable form for intraperitoneal, intravenous injection, or for injection at the tumor site.

The CD1d-independent NKT cells are preferably autologous to the patient.

The pharmaceutical composition is particularly useful for treating malignant tumors, i.e. cancers of any type. More particularly, the pharmaceutical composition is useful to slow down, limit or reverse growth of the tumor.

The tumor is an abnormal proliferation of cells, which can be malignant or not.

No antigenic restriction is needed, therefore any tumor can be treated.

In a preferred embodiment, tumor is a solid cancer. Examples of solid cancers include lung, kidney, bladder, liver, pancreas, colon, skin, brain, ovarian, cervical, and breast cancer.

In another embodiment, the tumor is a malignant hemopathy. In that case the CD1d-independent NKT cells are allogenous to the patient.

The below example illustrates the invention without limiting its scope.

### EXAMPLE

### 1. Capacity of nc-NKT to control tumor growth in a pancreatic adenocarcinoma model

**Methods** The inventors addressed the capacity of nc-NKT to control tumor growth and how tumor produced TGF-β controls nc-NKT cell homeostasis. Pancreatic Adenocarcinoma (PaCa) appears as a model of choice since enhanced expression of TGFβ correlates with tumor grade and decreased survival (Friess H, 1993). PaCa accounts for more than 85 % of pancreatic neoplasms. Despite of a rather low incidence (1/10 000) compared to other tumors, PaCa represents the forth leading cause of death by cancer. In humans, PaCa tumor progression is characterized by the appearance, inside pancreatic ducts, of precancerous dysplastic lesions of increasing grades, PanINs 1/2A/2B/3 (Pancreatic INtraepithelial Neoplasia.). These lesions precede the occurrence of malignant invasive tumor (PaCa). Recurrent mutations are observed all along the tumor progression process in humans (Bardeesy and Depinho 2002). Kras activating mutations are the first mutations detected. In about one third of low-grade PanINs and in virtually all PaCa. Ink4A/Arf loss of function mutations are described in the majority of mid-grade tumors (after Kras) and in 80-95% of PaCa (Aguirre et al, 2003). Kras activation within the pancreas (Cre-lox technology) of transgenic mice results in pre-cancerous lesions. Associated with inactivation of *Ink4A*/*ARF-KO,* Kras activation leads to the formation of aggressive malignant lesions (Aguirre, A. J., 2003,). In this work, the inventors used *Kras-CA* mutant mice as a model of PaCa precancerous lesions and *Kras-CA x Ink4A*/*ARF-KO* double mutant mice as a model of advanced PaCa cancerous lesions. The pancreatic localization of the tumors was performed by breeding either *Kras-CA*^{*lox*/}*^{lox}* animals or *Kras-CA*^{*lox*/*lox*} x *Ink4A*/*ARF*^{*lox*/*lox*} with Pdx-CRE animals. These models representative of the two stages of PaCa development, pre-malignant and established cancer, offer a powerful tool to analyze the anti-tumor activity of nc-NKT cells.

nc-NKT cells were obtained from mice deprived of TβR2 in T cells as described in Marie et al 2006. Briefly nc-NKT cells were either obtained from mice presenting a deletion of TβR2 specifically and selectively in T cells or by silencing *in vitro* TβR2 gene in naive T lymphocytes.

**Results (****fig. 1****)** The inventors reported that nc-NKT cells can kill cell line with a better efficacy that T lymphocyte counterparts. Because nc-NKT, exhibit hallmarks of NK cells, the inventors investigated whether the absence of MHC-I on cell leads to the activation of their cytotoxic functions like for NK cells. Their data demonstrate that similarly that of NK cells, nc-NKT cells can kill YAC-I cells (MHC-I^{-/-}). Thus cytotoxicity activity of nc-NKT cell involved TCR/MHC specific recognition used by CTLs and occurs also in the absence of MHC-I like for NK cells. Because of nc-NKT cells can kill cells MHC-I (^{-/-}), a common feature of numerous cancerous cells, inventors analyzed nc-NKT cell capacity to eliminate tumors in the organism. Purified nc-NKT cells or NK cells were injected ip in either 7-8 weeks old *Pdx-CRE x Kras-CA*^{*lox*/*lox*} animals or 3 week old *Pdx-CRE x Kras-CA*^{*lox*/}*^{lox} x Ink4A*/*ARF*^{*lox*/}*^{lox}* mice. Three weeks later histology analysis of pancreas was performed. Pre-cancerous PaCa lesions in *Pdx-CRE x Kras-CA*^{*lox*/*lox*} mice treated with nc-NKT were either not detectable, or slightly observed, while they remain in animals treated with NK cells. Surprisingly, in *Pdx-CRE x Kras-CA*^{*lox*/}*^{lox}* x *Ink4A*/*ARF* ^{*lox*/*lox*} mice injected with nc-NKT, established PaCa lesions were highly reduced compared to that of animals a treated with NK cells. These observations demonstrate the unique capacity of this herein described T cell subset, nc-NKT cells, to control tumor growth.

2. PKP16 cells were obtained by treatment of pancreatic adenocarcinoma isolated from PDX-CRE⁺ Kras^{+/-} P16^{-/-} mice of 3 weeks of age with collagenase and dispase and then cultured with RPMI 1640, supplemented with fetal calf serum 5%, L-Glutamine, and penicillin-streptomycin following classic cell culture conditions. At confluence cell culture was split. Cells after 20 splits, showing no presence of fibroblast, were used for experiments. 10⁶ PKP16 cells were co-cultured for 6 hours with different concentrations of ncNKT cells or T lymphocytes, and their survival monitored by flow cytometry using annexin V and 7-AAD staining. The results (**fig. 2**) show that ncNKT cells exhibit a high efficiency to kill tumoral cells straight derived from adenocarcinoma as compared to T lymphocytes.

### REFERENCES

- Aguirre AJ, Bardeesy N, Sinha M, Lopez L, Tuveson DA, Homer J, Redston MS, Depinho RA. Activated Kras and Ink4a/Arf deficiency cooperate to produce metastatic pancreatic ductal adenocarcinoma. Genes Dev, 2003, 17:3112-3126.
- Bardeesy N & Depinho RA. Pancreatic cancer biology and genetics. Nat.Rev.Cancer, 2002, 2:897-909.
- Chang, H., Brown, C. W., and Matzuk, M. M. (2002). Genetic analysis of the mammalian transforming growth factor-beta superfamily. Endocr Rev 23, 787-823.
- Derynck, R., and Zhang, Y. E. (2003). Smad-dependent and Smad-independent pathways in TGF-beta family signalling. Nature 425, 577-584.
- Friess, H., Yamanaka, Y., Buchler, M., Ebert, M., Beger, H. G., Gold, L. I., and Korc, M. (1993). Enhanced expression of transforming growth factor beta isoforms in pancreatic cancer correlates with decreased survival. Gastroenterology 105, 1846-1856.
- Gorelik, L., and Flavell, R. A. (2000). Abrogation of TGF-beta signaling in T cells leads to spontaneous T cell differentiation and autoimmune disease. Immunity 12, 171-181.
- He W, Dorn DC, Erdjument-Bromage H, Tempst P, Moore MA, Massague J. Hematopoiesis Controlled by Distinct TIF1 gamma and Smad4 Branches of the TGFbeta Pathway. Cell, 2006, 125:929-941
- Kaminska B, Wesolowska A, Danilkiewicz M. (2005). TGF beta signalling and its role in tumour pathogenesis. Acta Biochim Pol. 52(2):329-37.
- Letterio, J. J., and Roberts, A. B. (1998). Regulation of immune responses by TGF-beta. Annu Rev Immunol 16, 137-161.
- Levy L & Hill CS. Smad4 dependency defines two classes of transforming growth factor beta (TGF-β) target genes and distinguishes TGF-β-induced epithelial-mesenchymal transition from its antiproliferative and migratory responses. Mol Cell Biol, 2005, 25:8108-8125.
- Marie, J. C., Letterio, J. J., Gavin, M., and Rudensky, A. Y. (2005). TGF-betal maintains suppressor function and Foxp3 expression in CD4+CD25+ regulatory T cells. J Exp Med 201, 1061-1067.
- Marie JC, Liggitt D, Rudensky AY(2006). Cellular mechanisms of fatal early-onset autoimmunity in mice with the T cell-specific targeting of transforming growth factor-beta receptor. Immunity. 25(3):441-54.
- Massague, J., Andres, J., Attisano, L., Cheifetz, S., Lopez-Casillas, F., Ohtsuki, M., and Wrana, J. L. (1992). TGF-beta receptors. Mol Reprod Dev 32, 99-104.
- Muller AJ, Scherle PA (2006). Nat Rev Cancer. 6(8):613-25.
   Targeting the mechanisms of tumoral immune tolerance with small-molecule inhibitors..
- Thomas, D. A., and Massague, J. (2005). TGF-beta directly targets cytotoxic T cell functions during tumor evasion of immune surveillance. Cancer Cell 8, 369-380.

## Claims

1. Use of CD1d-independent NKT cells, for the preparation of a pharmaceutical composition for treating a tumor in a patient.

2. The use of claim 1, wherein said CD1d-independent NKT cells express FasL, perforin, granzymes and IFN-γ.

3. The use of claim 1 or 2, wherein said CD1d-independent NKT cells are obtained by
a. culturing a population of T cells under conditions which suppress TGFβ signalling pathway, and
b. selecting the cells which exhibit at least one NK marker.

4. The use of claim 3, wherein the population of T cells in (a) are cultured in the presence of an anti-TGFβ antibody.

5. The use of claim 3, wherein the population ofT cells in (a) are cultured in the presence of a TGFβ inhibitor.

6. The use of claim 3, wherein the NK marker is selected from the group consisting of. NKG2A, NKG2C, NKG2E, NKG2D, CD94, and DX5.

7. The use of any of claims 1 to 6, wherein said CD1d-independent NKT cells are autologous to the patient.

8. The use of any of claims 1 to 7, wherein the pharmaceutical composition is suitable for intraperitoneal or intravenous injection.

9. The use of any of claims 1 to 8, wherein the tumor is a solid cancer.

10. The use of claim 9, wherein the tumor is selected from the group consisting of lung, kidney, bladder, liver, pancreas, colon, skin, ovarian, cervical, and breast cancer.

11. The use of claim 1, wherein the tumor is a malignant hemopathy, and said CD1d-independent NKT cells are allogenous to the patient.

12. The use of any of claims 1 to 11, wherein the pharmaceutical composition limits or reverses tumor growth.

## Patentansprüche

1. Verwendung von CD1d-unabhängigen NKT-Zellen für die Herstellung einer pharmazeutischen Zusammensetzung zur Behandlung eines Tumors in einem Patienten.

2. Verwendung nach Anspruch 1, wobei die CD1d-unabhängigen NKT-Zellen FasL, Perforin, Granzyme und IFN-γ exprimieren.

3. Verwendung nach Anspruch 1 oder 2, wobei die CD1d-unabhängigen NKT-Zellen erhalten werden durch:
a. Züchten einer Population von T-Zellen unter Bedingungen, welche den TGF-β-Signalweg supprimieren, und
b. Selektieren der Zellen, welche mindestens einen NK-Marker aufweisen.

4. Verwendung nach Anspruch 3, wobei die Population von T-Zellen in (a) in Gegenwart eines Anti-TGF-β-Antikörpers gezüchtet wird.

5. Verwendung nach Anspruch 3, wobei die Population von T-Zellen in (a) in Gegenwart eines TGF-β-Inhibitors gezüchtet wird.

6. Verwendung nach Anspruch 3, wobei der NK-Marker aus der Gruppe, bestehend aus NKG2A, NKG2C, NKG2E, NKG2D, CD94 und DX5, ausgewählt ist.

7. Verwendung nach einem der Ansprüche 1 bis 6, wobei die CD1d-unabhängigen NKT-Zellen für den Patienten autolog sind.

8. Verwendung nach einem der Ansprüche 1 bis 7, wobei die pharmazeutische Zusammensetzung für die intraperitoneale oder intravenöse Injektion geeignet ist.

9. Verwendung nach einem der Ansprüche 1 bis 8, wobei der Tumor ein maligner Krebstumor ist.

10. Verwendung nach Anspruch 9, wobei der Tumor aus der Gruppe, bestehend aus Lungen-, Nieren-, Blasen-, Leber-, Bauchspeicheldrüsen-, Dickdarm-, Haut-, Eierstock-, Gebärmutterhals- und Brustkrebs, ausgewählt ist.

11. Verwendung nach Anspruch 1, wobei der Tumor eine maligne Bluterkrankung darstellt, und die CD1d-unabhängigen NKT-Zellen für den Patienten allogen sind.

12. Verwendung nach einem der Ansprüche 1 bis 11, wobei die pharmazeutische Zusammensetzung das Tumorwachstum begrenzt oder rückgängig macht.

## Revendications

1. Utilisation de cellules NKT indépendantes du CD1d, pour la préparation d'une composition pharmaceutique pour le traitement d'une tumeur chez un patient.

2. Utilisation selon la revendication 1, où lesdites cellules NKT indépendantes du CD1d expriment le FasL, la perforine, les granzymes et l'IFN-γ.

3. Utilisation selon la revendication 1 ou 2, où lesdites cellules NKT indépendantes du CD1d sont obtenues par
a. la culture d'une population de cellules T dans des conditions qui répriment la voie de signalisation du TGFβ, et
b. la sélection des cellules qui présentent au moins un marqueur NK.

4. Utilisation selon la revendication 3, où la population des cellules T en (a) est cultivée en présence d'un anticorps anti-TGFβ.

5. Utilisation selon la revendication 3, où la population des cellules T en (a) est cultivée en présence d'un inhibiteur du TGFβ.

6. Utilisation selon la revendication 3, où le marqueur NK est choisi dans le groupe comprenant NKG2A, NKG2C, NKG2E, NKG2D, CD94 et DX5.

7. Utilisation selon l'une quelconque des revendications 1 à 6, où lesdites cellules NKT indépendantes du CD1d sont autologues par rapport au patient.

8. Utilisation selon l'une quelconque des revendications 1 à 7, où la composition pharmaceutique est appropriée à une injection intrapéritonéale ou intraveineuse.

9. Utilisation selon l'une quelconque des revendications 1 à 8, où la tumeur est un cancer solide.

10. Utilisation selon la revendication 9, où la tumeur est choisie dans le groupe comprenant un cancer du poumon, du rein, de la vessie, du foie, du pancréas, du côlon, de la peau, ovarien, du col de l'utérus et du sein.

11. Utilisation selon la revendication 1, où la tumeur est une hémopathie maligne et lesdites cellules NKT indépendantes du CD1d sont allogéniques par rapport au patient.

12. Utilisation selon l'une quelconque des revendications 1 à 11, où la composition pharmaceutique limite ou inverse la croissance tumorale.
